# FASCICULE DE BREVET EUROPEEN

(11) **EP 1 000 604 B1**
(45) Date de publication et mention de la délivrance du brevet: **16.06.2004**
(21) Numéro de dépôt: 99402410.7
(22) Date de dépôt: 01.10.1999
(51) Int. Cl.: A61K 7/00

(54) **Composition cosmétique à phase continue hydrophile contenant du vanadate de bismuth**
Kosmetische Zusammensetzung mit einer Wismuthvanadat enthaltende kontinuierlicher hydophile phase
Continuous hydrophilic phase containing bismuth vanadate cosmetic composition

(30) Priorité: 10.11.1998 FR 9814159
(43) Date de publication de la demande: 17.05.2000
(73) Titulaire: L'OREAL, 75008 Paris (FR)
(72) Inventeur: Lemann, Patricia, 94000 Creteil (FR)
(74) Mandataire: Lhoste, Catherine

(56) Documents cités:
- EP-A- 0 632 110

## Description

La présente invention se rapporte à des compositions cosmétiques contenant un nouveau pigment jaune de couleur intense et saturée, non générateur de radicaux libres, et plus spécialement à des compositions de maquillage de la peau aussi bien du visage que du corps humain, des lèvres et des phanères comme les ongles, les cils, les sourcils ou les cheveux.

Les compositions de maquillage telles que les fonds de teint, les fards à joues ou à paupières, les rouges à lèvres, les produits anti-cernes, les blushes, les mascaras, les eye-liners, les crayons à lèvres ou à yeux ou encore les vernis à ongles et les produits de maquillage du corps, sont constituées d'un véhicule approprié et d'agents de coloration de natures différentes, destinés à conférer une certaine couleur à ces compositions, avant et/ou après leur application sur la peau, les lèvres et/ou les phanères.

Ces agents de coloration peuvent être des laques, des pigments minéraux ou organiques et/ou des pigments nacrés ou encore des colorants. Dans la gamme des pigments jaunes, les cosméticiens disposent de pigments d'origine minérale comme les oxydes de fer jaunes et de pigments d'origine organique. Les pigments minéraux, et en particulier les oxydes minéraux ont l'avantage d'être relativement stables mais ont l'inconvénient de donner des couleurs plutôt ternes et pâles. Les laques organiques ont l'avantage de conférer aux compositions des couleurs vives, mais sont pour la plupart instables à la lumière, à la température ou au pH. Certaines de ces laques présentent également l'inconvénient de tacher la peau ou les ongles de manière disgracieuse après application, par dégorgement du colorant. Les pigments nacrés quant à eux permettent d'obtenir des couleurs variées, mais jamais intenses, à effets irisés mais le plus souvent assez faibles.

Par ailleurs, certains agents de coloration présentent l'inconvénient d'engendrer des radicaux libres dans les formules de maquillage modifiant le rendu des couleurs et la stabilité des compositions, puis sur la peau après application, ce qui favorise le vieillissement cutané (apparition de rides, ridules, jaunissement de la peau). En particulier, les oxydes de fer jaune provoquent souvent une oxydation des huiles polyinsaturées (huiles végétales par exemple), ce qui limite la gamme de compositions. Comme agents de coloration présentant cet inconvénient ont peut citer notamment les mélanges d'oxydes de fer brun-jaune vendu sous la dénomination commerciale « Sicovit Jaune 10 E 172» par BASF par exemple, les pigments d'origine organique ; ainsi que la laque d'aluminium de tartrazine sur alumine (20/80) (CI :19140, CI: 77002) vendu sous le nom commercial FD & C Yellow 5 de Wamer Jenkinson.

Aujourd'hui pour pallier cet inconvénient, on utilise des agents anti-oxydants comme par exemple l'éthoxyquine. Malheureusement, il est souvent difficile de trouver un agent anti-oxydant efficace à 100% compte tenu de la multiplicité des ingrédients présents dans les compositions de maquillage. De plus, les agents anti-oxydants engendrent souvent eux-mêmes des produits de dégradation (oxydation de l'agent anti-oxydant) qui peuvent être gênants.

La présente invention a justement pour objet l'utilisation dans une composition cosmétique d'un nouveau pigment jaune de couleur intense et saturée, stable ayant l'avantage d'engendrer beaucoup moins de radicaux libres que les pigments classiquement utilisés notamment pour obtenir une couleur jaune.

De façon surprenante, le demandeur a trouvé que le vanadate de bismuth de formule BiVO₄ permettait de limiter la production de radicaux libres, puisqu'il a la propriété d'engendrer très peu de radicaux libres, et de limiter ainsi l'utilisation d'anti-oxydants dans les compositions. En outre ce pigment permet d'obtenir une coloration intense et en particulier un jaune citron, très vif et intense, de très grande pureté de couleur, non dégorgeante sur les matières kératiniques, stable à la lumière, au pH et à la température. Il permet, en outre, d'élargir la gamme de couleur dans le domaine cosmétique et de participer à la création de verts éclatants en mélange avec des bleus, d'oranges vifs. De plus, il permet de réduire la quantité de pigments de l'art antérieur, permettant ainsi de préserver la brillance de la composition ainsi que celle du film déposé, ce qui est très recherché pour les vernis à ongles et les produits de maquillage des lèvres.

De façon plus précise, l'invention pour objet une composition à application topique colorée à phase continue hydrophile, se présentant sous forme d'un produit de maquillage de la peau, des lèvres et/ou des phanères d'être humain, contenant du vanadate de bismuth (BiVO₄) comme agent de coloration et plus précisément de pigment. Il peut se présenter sous forme pure ou déposé sur un substrat. En particulier, il se présente sous forme pure comme celui commercialisé sous la référence Sicopal Gelb L1100 par la société BASF.

La fabrication de ce pigment est en particulier celle décrite dans les documents EP-A-551637, EP-A-632110 de BASF. Il présente en particulier une taille de 0,3 µm environ.

Le vanadate de bismuth peut cristalliser sous différentes formes et exprimer, ainsi des tons de jaunes différents :
- réseau monoclinique ; la fergusonite : jaune brillant, densité 6,959
- réseau tétragonal ; zircon : jaune pâle, densité 6,127
- réseau tétragonal ; scheelite : jaune brillant, densité 6,929
- réseau orthorhombique ; pulchérite : jaune pâle.

Pour montrer la propriété qu'a le vanadate de bismuth de ne pas engendrer de radicaux libres, le demandeur a réalisé un test d'éthylène selon le procédé décrit dans l'article « Ethylene formation from methionine as a method to evaluate oxygen free radical scavenging and metal inactivation by cosmetics » de J.-B. Galey, F. Millecamps et Q.-L. Nguyen, *International Journal of Cosmetic Science,* 13, 65-78, 1991.

L'objectif est de comparer le comportement du pigment minéral selon l'invention, par rapport à celui de pigments classiques dans un test de photo-oxydation utilisant le fer comme générateur de radicaux libres.

Dans le protocole de mesure du test éthylène, le FeCl₃ utilisé pour activer la production de radicaux libres a été substitué par chacun des pigments à tester. Les résultats sont donnés dans le tableau ci-après.

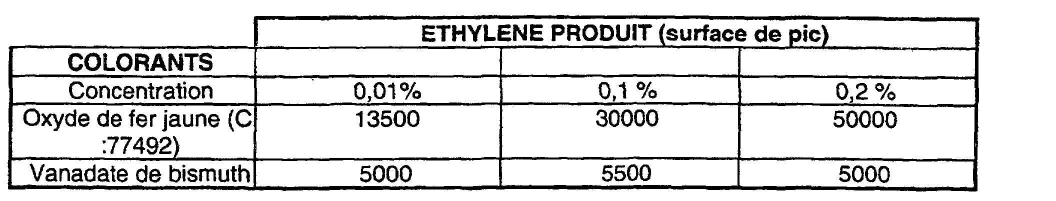

Le témoin FeCl3 à 0,005 % étant en moyenne à 9000 (unité arbitraire - mesure relative).

Plus la quantité d'éthylène est élevée, plus la production de radicaux libres est élevée.

L'oxyde de fer jaune ( Cl : 77492) n'est pas inerte. A faible dose, il active jusqu'à une certaine concentration où l'effet protecteur du pigment commence à jouer, tandis que pour le vanadate de bismuth, le taux d'éthylène produit est très faible et n'évolue pas en fonction de la concentration. Ce pigment pourra donc être avantageusement utilisé dans des compositions de maquillage et des compositions colorées solaires notamment destinées à la protection de la peau et/des muqueuses telles que les lèvres, sans engendrer de radicaux libres et donc limiter ainsi la dégradation de la peau et/ou des muqueuses.

Le pigment de la composition de l'invention présente, en outre, par rapport aux oxydes de fer jaunes couramment utilisés en cosmétique, l'avantage d'être plus saturé en couleur, d'être plus vif, de couleur plus intense, ce qui permet, notamment de l'utiliser en plus faible quantité, pour un rendu de couleur équivalent. On donne ci-après les paramètres colorimétriques de vanadate de bismuth vendu par la société BASF sous la marque commerciale Sicopal Gelb L 1100 par rapport à celui de l'oxyde de fer jaune (CI: 77492).

| Paramètres | Oxyde de fer jaune | vanadate de bismuth |
|---|---|---|
| L | 69,9 | 92,8 |
| a | 6,85 | -12,92 |
| b | 50,35 | 84,64 |
| c | 50,6 | 85,27 |

Plus la valeur de c est élevée, plus la couleur est saturée : le vanadate de bismuth a une couleur plus vive, plus intense que l'oxyde de fer, ce qui permet de colorer fortement une composition cosmétique avec une faible quantité de pigment. Ainsi les problèmes de rhéologie (difficulté d'application, maquillage hétérogène) liés à une trop forte quantité de pigments dans les compositions de l'art antérieur sont largement atténués.

Par rapport à une laque organique, le vanadate de bismuth est plus couvrant, à quantité de pigment égale.

Le pigment selon de l'invention peut être incorporé dans une composition cosmétique à phase continue hydrophile notamment de maquillage en une quantité déterminable aisément par l'homme du métier sur la base de ses connaissances générales, et qui peut notamment aller de 0,01 à 50% en poids par rapport au poids de la composition, de préférence en une quantité allant de 0,5 à 25% en poids. Même à concentration élevée, ce pigment ne déstructure pas la composition.

La composition de l'invention se présente sous forme d'un produit à appliquer sur les lèvres, les yeux, la peau et/ou les phanères d'êtres humains. Elle contient donc un milieu cosmétiquement acceptable, c'est-à-dire un milieu compatible avec toutes les matières kératiniques telles que la peau aussi bien du corps humain que du visage, les ongles, les cheveux, les cils et sourcils.

Selon l'invention, ce milieu contient une phase continue hydrophile, c'est-à-dire un mélange d'un ou plusieurs corps hydrophiles, miscibles ou solubles au moins en partie avec l'eau, qui peuvent être liquides, pâteux ou solides à température ambiante (25°C en général). En particulier, ce milieu peut comprendre ou se présenter notamment sous forme de suspension, dispersion ou solution dans l'eau ou un milieu hydroalcoolique, éventuellement épaissi, voire gélifié ; émulsion huile-dans-eau (H/E), ou multiple (E/H/E), sous forme de crème, de pâte ou même de solide ; gel aqueux ou hydroalcoolique ou mousse hydrophile ; gel émulsionné ; dispersion de vésicules notamment de lipides ioniques ou non ; lotion biphase ou multiphase ; spray. L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition comprend donc une phase continue hydrophile qui peut contenir de l'eau ou un mélange d'eau et de solvant organiques hydrophiles comme les alcools et notamment des monoalcools inférieurs linéaires ou ramifiés ayant de 2 à 5 atomes de carbone comme l'éthanol ou le propanol, les polyols comme la glycérine, la diglycérine, le propylène glycol, le sorbitol, le panthénol, le pentylène glycol, les polyéthylène glycols. Cette phase continue peut représenter de 0,5 à 99,99% du poids total de la composition. Elle peut, en outre, contenir des éthers en C₂ et des aldéhydes en C₂-C₄ hydrophiles.

Lorsque la composition selon l'invention se présente sous la forme d'une émulsion, elle peut éventuellement comprendre, en outre, un tensioactif, de préférence en une quantité de 0 à 30% et notamment de 0,01 à 30% en poids par rapport au poids total de la composition.

Selon l'application envisagée, la composition peut comprendre, en outre, un polymère filmogène (comme les polyuréthannes, les polyacryliques, les hybrides polyuréthannes et polyacryliques, les polyesters, la nitrocellulose, les résines hydrocarbonées et/ou siliconées). Ceci est notamment le cas lorsqu'on souhaite préparer une composition de type vernis à ongles à milieu aqueux, mascara, eye-liner ou composition capillaire de type laque. Les polymères peuvent être dissous ou dispersés dans le milieu cosmétiquement acceptable et éventuellement associés à des agents de coalescente et/ou des plastifiants.

La composition selon l'invention peut également comprendre une phase grasse, notamment constituée de corps gras liquides à température ambiante (25°C en général) et/ou de corps gras solides à température ambiante tels que les cires, les corps gras pâteux, les gommes et leurs mélanges. Cette phase grasse peut, en outre, contenir des solvants organiques lipophiles.

Comme corps gras liquides à température ambiante, appelés souvent huiles, utilisables dans l'invention, on peut citer : les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycé des des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, palmitate d'éthyl-2hexyle, stéarate d'octyl-2-dodécyle, érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

Ces huiles peuvent représenter de 0 à 90 %, et mieux de 0 à 85 % en poids par rapport au poids total de la phase lipophile.

La phase lipophile de la composition selon l'invention peut également comprendre un ou plusieurs solvants organiques, cosmétiquement acceptables (tolérance, toxicologie et toucher acceptables). Ces solvants peuvent représenter de 0 à 60 % et mieux de 0 à 30% du poids total de la composition et peuvent être choisis dans le groupe constitué par les solvants organiques lipophiles, les solvants amphiphiles ou leurs mélanges. Comme solvants utilisables dans la composition de l'invention, on peut citer les esters de l'acide acétique comme l'acétate de méthyle, d'éthyle, de butyle, d'amyle, de méthoxy-2-éthyle ; les cétones comme la méthyléthylcétone, la méthylisobutylcétone, l'acétate d'isopropyle ; les hydrocarbures comme le toluène, le xylène, le p-cylène, l'hexane, l'heptane ; les aldéhydes ayant de 5 à 10 atomes de carbone ; les éthers ayant au moins 3 atomes de carbones ; et leurs mélanges.

La composition selon l'invention peut, de plus, comprendre tous les ingrédients classiquement utilisés dans les domaines concernés et plus spécialement dans les domaines cosmétique et dermatologique. Ces ingrédients sont en particulier choisis parmi les conservateurs, les épaississants de phase aqueuse (biopolymères polysaccharidiques, les polymères synthétiques) ou grasse, les parfums, les actifs hydrophiles ou lipophiles et leurs mélanges. Les quantités de ces différents ingrédients sont celles classiquement utilisées dans les domaines concernés et par exemple de 0,01 % à 20 % du poids total de la composition. La nature de ces ingrédients et leur proportion doit être compatibles avec l'obtention de compositions selon l'invention, stables épaissies et brillantes.

La composition de l'invention peut, en outre, comprendre une phase particulaire additionnelle pouvant être présente à raison de 0 à 30 % du poids total de la composition, de préférence de 0,05 à 20 % et qui peut comprendre des pigments et/ou des nacres et/ou des charges utilisés dans les compositions cosmétiques.

Par pigments, il faut comprendre des particules blanches ou colorées, minérales ou organiques, insolubles dans la phase hydrophile liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments autres que le vanadate de bismuth peuvent être présents dans la composition à raison de 0 à 25 % du poids de la composition finale, et de préférence à raison de 2 à 15 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium, aluminium ou encore les dicéto pyrrolopyrrole (DPP) décrits dans les documents EP-A-542669, EP-A-787730, EP-A-787731 et WO 96/08537.

Les nacres peuvent être présentes dans la composition à raison de 0 à 20 % du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 30 % du poids total de la composition, de préférence 0,5 à 15 %. On peut notamment citer le talc, le stéarate de zinc, le mica, le kaolin, les poudres de Nylon (Orgasol notamment) et de polyéthylène, le Téflon, l'amidon, le nitrure de bore, des microsphères de copolymères telles que l'Expancel (Nobel Industrie), le polytrap (Dow Corning) et les microbilles de résine de silicone (Tospearl de Toshiba, par exemple).

La composition de l'invention peut comprendre, avantageusement une phase grasse solide ou pâteuse, contenant une ou plusieurs gommes et/ ou une ou plusieurs cires. Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45°C.

Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les gommes sont généralement des PDMS à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0 à 50 % en poids de cires, par rapport au poids total de la composition et mieux de 1 à 30 %.

Cette composition peut avoir l'aspect d'une crème, pommade, lotion fluide, de pâte souple de viscosité dynamique à 25°C de l'ordre de 1 à 40 Pa.s, onguent, solide coulé ou moulé et notamment en stick ou en coupelle.

La composition selon l'invention peut être avantageusement utilisée pour le maquillage de la peau et/ou des lèvres et/ou des phanères selon la nature des constituants utilisés. En particulier, cette composition peut être un bâton de rouge à lèvres ou une laque à lèvres, un brillant à lèvres (gloss en terminologie anglo-saxonne) utilisable tel quel ou pour appliquer sur un film de rouge à lèvres notamment pour en augmenter sa brillance et/ou sa couleur (top coat en terminologie anglo-saxonne). Elle peut aussi constituer un fond de teint solide, un produit anti-cernes ou contours des yeux, un eye liner, un mascara, une ombre à paupières, un blush, un vernis à ongles. Ces compositions peuvent, en outre, contenir des actifs cosmétiques ou dermatologiques, en vue, notamment d'apporter un aspect soin ou traitant à la composition. Ainsi la composition peut contenir des vitamines et autres actifs lipophiles (lanoline, filtre UVA) ou hydrophiles (hydratants comme la glycérine).

L'invention a encore pour objet une utilisation cosmétique de la composition ci-dessus pour soigner et/ou maquiller et/ou protéger la peau et/ou les lèvres et/ou les phanères d'êtres humains ainsi qu'une utilisation de cette composition pour la préparation d'un onguent destiné à traiter et/ou protéger la peau et/ou les lèvres et/ou les phanères. L'invention a aussi pour objet un procédé de traitement cosmétique de la peau et/ou des lèvres et/ou des phanères, consistant à appliquer sur la peau et/ou les lèvres et/ou les phanères la composition définie ci-dessus.

De façon plus spécifique, l'invention a pour objet un produit à lèvres, un fond de teint ou un vernis à ongle.

La composition de l'invention peut être obtenue selon les procédés de préparation classiquement utilisés en cosmétique ou en dermatologie.

L'invention a encore pour objet l'utilisation, dans une composition cosmétique ou pour la fabrication d'une composition dermatologique, colorée, d'un agent de coloration tel que décrit ci-dessus, afin de protéger la peau et/ou les lèvres et/ou les phanères contre les méfaits des radicaux libres et/ou lutter contre les signes cutanés du vieillissement notamment photoinduit. Ces signes du vieillissement sont en particulier les rides, ridules, la peau flasque et/ou jaunie.

L'invention a encore pour objet un procédé de protection cosmétique de la peau et/ou des lèvres et/ou des phanères, contre les méfaits des radicaux libres et/ou de lutte contre les signes cutanés du vieillissement photoinduit, consistant à appliquer sur la peau et/ou les lèvres et/ou les phanères la composition telle que définie ci-dessus.

Les exemples de compositions ci-après sont donnés à titre illustratif et sans caractère limitatif. Les pourcentages sont des pourcentages en poids.

### Exemple 1 : de fond de teint de type H/E

- *Phase A*
   . Acide stéarique 2,0 %
   . Stéarate de glycéryle 3,0 %
   . Isostéarate de glycéryle 2,0 %
   . Huile minérale 8,0 %
   . Pigments
      Oxyde de fer rouge 0,9 %
      Oxyde de fer noir 0,3%
      Dioxyde de titane 4,4 %
      Vanadate de bismuth 0,7 %
   . Conservateur 0,2 %
   . Diméthicone (5cst) à vérifier 4,0 %
- *Phase B*
   Triéthanolamine 1,0 %
- *Phase C*
   . Conservateur 0,2 %
   . Silicate de magnésium et d'aluminium
      en gel à 5% de matière active 20,0%
   . Gomme de cellulose 3,5 %
   . Lauroyl sarcosinate de sodium 3,5 %
   . Glycérine 2,0 %
   . Eau qsp 100 %
- *Phase D*
   Conservateur 0,3 %
   Eau 2,0 %

- *Mode opératoire:*
   On prépare les phases A et C séparément, on les chauffe à 80°C puis on les homogénéise à l'homogénéisateur commercialisé sous le nom de Moritz. On introduit la phase B dans la phase A, puis on verse ce mélange dans la phase C sous agitation. On ajoute ensuite la phase D et on poursuit l'agitation jusqu'à un complet refroidissement.
   On obtient un fond de teint de couleur beige, de couvrance élevée, stable à la lumière, ne laissant aucune griffe (ou marque) après démaquillage.

### Exemple 2 : de laque à lèvres

- *Composition*
   . Dispersion aqueuse de polymère acrylique/styrène
      (Néocryl A-1052 de Zeneca) 20,0 % matière active
   . Acétyltributylcitrate 2,5 %
   . Vanadate de bismuth 1,5%
   . Rouge Flaming (Cl 12085) 1,5 %
   . Glycérine 1,25 %
   . Eau qsp 100%
- *Mode opératoire:*
   On ajoute à température ambiante l'acétyltributylcitrate, les pigments ; la phase aqueuse (glycérine + eau) à la dispersion de polymère puis on homogénéise.
   On obtient une laque à lèvres de couleur orange, stable et couvrante.

## Revendications

1. Composition à application topique colorée à phase continue hydrophile, se présentant sous forme d'un produit de maquillage de la peau, des lèvres et/ou des phanères d'être humain, comprenant du vanadate de bismuth comme agent de coloration.

2. Composition selon la revendication 1, **caractérisée en ce que** l'agent de coloration est présent à raison de 0,01 à 50% en poids par rapport au poids de la composition.

3. Composition selon la revendication 1 ou 2, **caractérisée en ce que** l'agent de coloration est présent à raison de 0,5 à 25% en poids par rapport au poids de la composition.

4. Composition selon l'une des revendications précédentes, se présentant sous forme d'un vernis à ongles, mascara, eye-liner ou composition capillaire de type laque, produit à lèvres, brillant à lèvres, fond de teint, produit anti-cernes, fard à joues ou à paupières, maquillage du corps.

5. Composition selon l'une des revendications précédentes, **caractérisée en ce que** la phase continue hydrophile contient de l'eau, un mélange d'eau et de solvants hydrophiles.

6. Composition selon l'une des revendications précédentes, comprenant, en outre, au moins une phase grasse choisie parmi les huiles, les cires, les gommes, les corps gras pâteux, les solvants organiques lipophiles et leurs mélanges.

7. Composition selon l'une des revendications précédentes, **caractérisée en ce qu'**elle contient, en outre, une phase particulaire additionnelle pouvant être présente à raison de 0 à 30 % du poids total de la composition.

8. Composition selon l'une des revendications précédentes, se présentant sous forme d'émulsion H/E ou de dispersion de polymère en milieu aqueux ou hydroalcoolique, de gel.

9. Utilisation cosmétique de la composition selon l'une des revendications précédentes, pour soigner et/ou maquiller et/ou protéger la peau et/ou les lèvres et/ou les phanères.

10. Utilisation de la composition selon l'une des revendications 1 à 8 pour la préparation d'un onguent destiné à traiter et/ou protéger la peau et/ou les lèvres et/ou les phanères.

11. Utilisation dans une composition cosmétique colorée ou pour la fabrication d'une composition dermatologique, colorée, de vanadate de bismuth en vue de protéger la peau et/ou les lèvres et/ou les phanères contre les méfaits des radicaux libres et/ou lutter contre les signes cutanés du vieillissement.

12. Procédé de protection cosmétique de la peau et/ou des lèvres et/ou des phanères, contre les méfaits des radicaux libres et/ou de lutte contre les signes cutanés du vieillissement photoinduit, consistant à appliquer sur la peau et/ou les lèvres et/ou les phanères la composition selon l'une des revendications 1 à 8.

## Patentansprüche

1. Farbige Zusammensetzung zur topischen Anwendung mit einer kontinuierlichen hydrophilen Phase, die als Produkt zum Schminken der menschlichen Haut, der Lippen und/oder der Hautanhangsgebilde vorliegt und die als Farbmittel Bismutvanadat enthält.

2. Zusammensetzung nach Anspruch 1, **dadurch gekennzeichnet, dass** das Farbmittel in einer Menge von 0,01 bis 50 Gew.-%, bezogen auf das Gewicht der Zusammensetzung, vorliegt.

3. Zusammensetzung nach Anspruch 1 oder 2, **dadurch gekennzeichnet, dass** das Farbmittel in einer Menge von 0,5 bis 25 Gew.-%, bezogen auf das Gewicht der Zusammensetzung, enthalten ist.

4. Zusammensetzung nach einem der vorhergehenden Ansprüche, die als Nagellack, Mascara, Eyeliner, Zusammensetzung für das Haar vom Typ Lack, Produkt für die Lippen, Lippenglanz, Makeup, Produkt zum Abdecken von Augenringen, Wangenrouge, Lidschatten oder Schminkprodukt für den Körper vorliegt.

5. Zusammensetzung nach einem der vorhergehenden Ansprüche, **dadurch gekennzeichnet, dass** die kontinuierliche hydrophile Phase Wasser oder ein Gemisch von Wasser und hydrophilen Lösungsmitteln enthält.

6. Zusammensetzung nach einem der vorhergehenden Ansprüche, die ferner mindestens eine Fettphase enthält, die unter den Ölen, Wachsen, Gummis, pastösen Fettsubstanzen, lipophilen organischen Lösungsmitteln und deren Gemischen ausgewählt ist.

7. Zusammensetzung nach einem der vorhergehenden Ansprüche, **dadurch gekennzeichnet, dass** sie außerdem eine zusätzliche Partikelphase enthält, die in einer Menge von 0 bis 30 % des Gesamtgewichts der Zusammensetzung vorliegen kann.

8. Zusammensetzung nach einem der vorhergehenden Ansprüche, die als Emulsion O/W, als Polymerdispersion in einem wässrigen oder wässrig-alkoholischen Medium oder als Gel vorliegt.

9. Kosmetische Verwendung der Zusammensetzung nach einem der vorhergehenden Ansprüche zur Pflege und/oder zum Schinken und/oder zum Schutz der Haut und/oder der Lippen und/oder der Hautanhangsgebilde.

10. Verwendung einer Zusammensetzung nach einem der Ansprüche 1 bis 8 zur Herstellung einer Salbe, die zur Behandlung und/oder zum Schutz der Haut und/oder der Lippen und/oder der Hautanhangsgebilde vorgesehen ist.

11. Verwendung von Bismutvanadat in einer farbigen kosmetischen Zusammensetzung oder zur Herstellung einer farbigen dermatologischen Zusammensetzung zum Schutz der Haut und/oder der Lippen und / oder der Hautanhangsgebilde gegen die schädlichen Wirkungen von freien Radikalen und/oder zur Bekämpfung der Anzeichen der Hautalterung.

12. Kosmetisches Verfahren zum Schutz der Haut und/oder der Lippen und/oder der Hautanhangsgebilde gegen die schädlichen Wirkungen von freien Radikalen und/oder zur Bekämpfung der Anzeichen der lichtinduzierten Hautalterung, das darin besteht, auf die Haut und/oder die Lippen und/oder die Hautanhangsgebilde eine Zusammensetzung nach einem der Ansprüche 1 bis 8 aufzutragen.

## Claims

1. Coloured composition for topical application, containing a hydrophilic continuous phase, which is in the form of a make-up product for the skin, the lips and/or the superficial body growths of a human being, and comprises bismuth vanadate as colouring agent.

2. Composition according to Claim 1, **characterized in that** the colouring agent is present in a proportion of from 0.01 to 50% by weight relative to the weight of the composition.

3. Composition according to Claim 1 or 2, **characterized in that** the colouring agent is present in a proportion of from 0.5 to 25% by weight relative to the weight of the composition.

4. Composition according to one of the preceding claims, which is in the form of a nail varnish, mascara, eyeliner, hair composition such as a lacquer, lip product, lip gloss, foundation, concealer product, face powder, eyeshadow or body make-up.

5. Composition according to one of the preceding claims, **characterized in that** the hydrophilic continuous phase contains water or a mixture of water and hydrophilic solvents.

6. Composition according to one of the preceding claims, also comprising at least one fatty phase chosen from oils, waxes, gums, pasty fatty substances, lipophilic organic solvents and mixtures thereof.

7. Composition according to one of the preceding claims, **characterized in that** it also contains an additional particulate phase which can be present in a proportion of from 0 to 30% of the total weight of the composition.

8. Composition according to one of the preceding claims, which is in the form of an O/W emulsion or a dispersion of polymer in an aqueous or aqueous-alcoholic medium, or in the form of a gel.

9. Cosmetic use of the composition according to one of the preceding claims, to care for and/or make up and/or protect the skin and/or the lips and/or the superficial body growths.

10. Use of the composition according to one of Claims 1 to 8 for the preparation of an ointment intended to treat and/or protect the skin and/or the lips and/or the superficial body growths.

11. Use, in a coloured cosmetic composition or for the manufacture of a coloured dermatological composition, of bismuth vanadate in order to protect the skin and/or the lips and/or the superficial body growths against the harmful effects of free radicals and/or to combat the signs of ageing of the skin.

12. Process for the cosmetic protection of the skin and/or the lips and/or the superficial body growths against the harmful effects of free radicals and/or for combating the signs of photo-induced ageing of the skin, which consists in applying the composition according to one of Claims 1 to 8 to the skin and/or the lips and/or the superficial body growths.
